# EUROPEAN PATENT APPLICATION

(11) **EP 4 664 106 A1**
(43) Date of publication of application: **17.12.2025**
(21) Application number: 24753388.8
(22) Date of filing: 07.02.2024
(51) Int. Cl.: G01N 33/576, G01N 33/543

(54) **SENSITIZER FOR IMMUNOCHROMATOGRAPHIC MEASUREMENTS TARGETING HEPATITIS VIRUS, SPECIMEN DILUENT FOR IMMUNOCHROMATOGRAPHIC MEASUREMENTS, IMMUNOCHROMATOGRAPHIC MEASUREMENT METHOD, TOOL FOR IMMUNOCHROMATOGRAPHIC MEASUREMENTS AND TEST KIT FOR HEPATITIS VIRUS**

(30) Priority: 09.02.2023 JP 2023018698
(71) Applicant: Kawasaki Gakuen Educational Foundation, Kurashiki-shi, Okayama 701-0192 (JP); NOF Corporation, Shibuya-ku Tokyo 150-6019 (JP)
(72) Inventor: NAKAHARA, Takako, Kurashiki-shi, Okayama 701-0193 (JP); MURAKAMI, Keiji, Kurashiki-shi, Okayama 701-0193 (JP); KOBAYASHI, Koh, Kawasaki-shi, Kanagawa 210-0865 (JP); MATSUDA, Masaru, Kawasaki-shi, Kanagawa 210-0865 (JP); HARADA, Eiji, Kawasaki-shi, Kanagawa 210-0865 (JP)
(74) Representative: Weickmann & Weickmann PartmbB
(86) International application number: PCT/JP2024/004110
(87) International publication number: WO 2024/166945

(57) **Abstract**

Provided is a sensitizer for immunochromatographic assay that can detect hepatitis virus with high sensitivity, and an assay using the sensitizer, and the like.

A sensitizer for immunochromatographic assay of hepatitis virus as an assay target substance, comprising a polymer represented by the following general formula [1]: 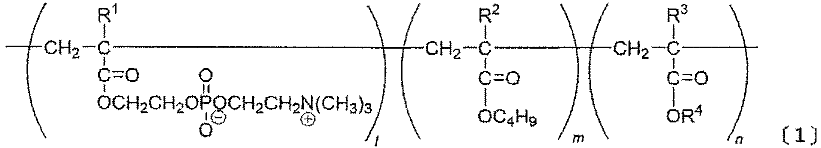 wherein R¹, R² and R³ are the same or different and each is a hydrogen atom or a methyl group, R⁴ is a hydrogen atom, a cation or a 2,3-dihydroxypropyl group, and 1, m and n are each a constitutional unit number and l:m:n is 20 to 100:0 to 80:0 to 80.

## Description

### [Technical Field]

The present invention relates to a sensitizer for immunochromatographic assay of hepatitis virus as an assay target substance, a sample diluent for immunochromatographic assay, immunochromatographic assay, an instrument for immunochromatographic assay, and a test kit for hepatitis B virus.

### [Background Art]

Immunochromatographic assay is a method that combines a chromatographic method using membrane capillarity and an immunological method using an antigen-antibody reaction. Immunochromatography is widely used as a clinical diagnosis method because it has high specificity due to the antigen-antibody reaction, is easy to operate, can be tested on the spot without requiring a place or special facility, and permits visual observation of the results (see, for example, Patent Literature 1).

Immunochromatographic assay is advantageous in that the assay is easy, but the accuracy of detection sensitivity is low, and positive samples are often judged as false negatives. Thus, an immunochromatographic assay with higher sensitivity is desired.

To date, various methods have been studied for the purpose of establishing a highly sensitive immunochromatographic assay. For example, Patent Literature 2 discloses a method of increasing sensitivity by changing the addition position of immunochromatographic reagents.

Furthermore, a method of increasing sensitivity by adding an additive to sample diluents used in conventional immunochromatographic assay is known. For example, addition of hyaluronic acid (Patent Literature 3) and a polymer having a phosphorylcholine group (Patent Literatures 4 and 5) to sample diluents are disclosed. These disclosures are advantageous in that the addition position of reagents does not need to be changed and that conventional membranes for immunochromatography can be used.

### [Citation List]

### [Patent Literature]

[Patent Literature 1]
   JP-A-H01-063865
[Patent Literature 2]
   JP-A-2014-66674
[Patent Literature 3]
   JP-A-2008-292326
[Patent Literature 4]
   JP-A-2008-058334
[Patent Literature 5]
   JP-A-2003-344413

### [Summary of Invention]

### [Technical Problem]

However, in the above-mentioned Patent Literatures 3 to 5, the assay targets are influenza virus, human C-reactive protein (CRP) and syphilis antigen, but hepatitis virus is not considered as an assay target. In other words, there is a demand for a sensitizer that can be used in a method for assaying hepatitis virus with high sensitivity.

The present invention aims to provide a sensitizer for immunochromatographic assay that can detect hepatitis virus with high sensitivity, a sample diluent for immunochromatographic assay, immunochromatographic assay, an instrument for immunochromatographic assay, and a test kit for hepatitis B virus.

### [Solution to Problem]

The present inventors have conducted intensive studies in an attempt to find a compound capable of detecting hepatitis virus with high sensitivity and found that a copolymer represented by the following general formula [1] and having a group having a phosphorylcholine group as a side chain has the desired properties, which resulted in the completion of the present invention.

That is, the present invention relates to the following.
[1] A sensitizer for immunochromatographic assay of hepatitis virus as an assay target substance, comprising a polymer represented by the following general formula [1]: 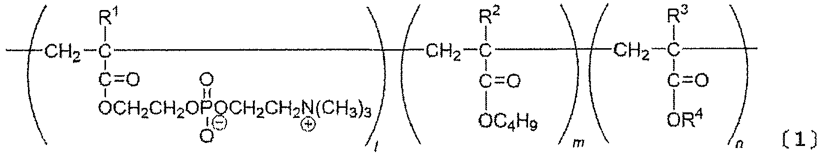 wherein R¹, R² and R³ are the same or different and each is a hydrogen atom or a methyl group, R⁴ is a hydrogen atom, a cation or a 2,3-dihydroxypropyl group, and 1, m and n are each a constitutional unit number and l:m:n is 20 to 100:0 to 80:0 to 80.
[2] A sample diluent for immunochromatographic assay of hepatitis virus as an assay target substance, comprising the sensitizer for immunochromatographic assay of the above-mentioned [1].
[3] A method for immunochromatographically assaying hepatitis virus as an assay target substance, comprising conducting an antigen-antibody reaction in the presence of the sensitizer for immunochromatographic assay of the above-mentioned [1].
[4] An instrument for immunochromatographic assay of hepatitis virus as an assay target substance, comprising the sensitizer for immunochromatographic assay of the above-mentioned [1].
[5] A test kit for hepatitis virus, comprising a polymer represented by the following general formula [1] and a substance that specifically binds to hepatitis virus: 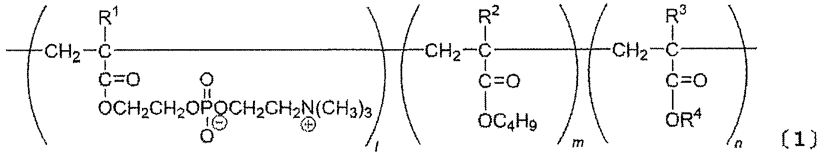 wherein R¹, R² and R³ are the same or different and each is a hydrogen atom or a methyl group, R⁴ is a hydrogen atom, a cation or a 2,3-dihydroxypropyl group, and 1, m and n are each a constitutional unit number and l:m:n is 20 to 100:0 to 80:0 to 80.

### [Advantageous Effects of Invention]

According to the present invention, it is possible to provide a sensitizer for immunochromatographic assay that can detect hepatitis virus with high sensitivity, and an assay using the sensitizer, and an assay instrument and a test kit for hepatitis virus, each containing the sensitizer.

### [Description of Embodiments]

A sensitizer for immunochromatographic assay of hepatitis virus as an assay target substance, a sample diluent containing the sensitizer which is for immunochromatographic assay of hepatitis virus as an assay target substance, a method for immunochromatographically assaying hepatitis virus as an assay target substance, including conducting an antigen-antibody reaction in the presence of the sensitizer, an instrument containing the sensitizer which is for immunochromatographic assay of hepatitis virus as an assay target substance, and a test kit containing the sensitizer which is for hepatitis virus, of the present invention are described below.

In a method using the sensitizer for immunochromatographic assay of the present invention, the assay target substance is hepatitis virus, but other assay target substances can also be detected. The sensitizer for immunochromatographic assay of the present invention is characterized in that it can detect the above-mentioned virus with high sensitivity.

In the present specification, hepatitis viruses refer to hepatitis viruses such as hepatitis A, B, C, D, and E viruses. Hepatitis A and E viruses are mainly transmitted through food, while hepatitis B, C, and D viruses are mainly transmitted through blood. In particular, infection with types B and C of hepatitis virus can cause chronic liver disease. Hepatitis can cause the liver to function poorly, leading to symptoms such as fatigue, loss of appetite, and nausea. Therefore, the type of hepatitis virus as an assay target substance in the present invention is not particularly limited, but is preferably type B or type C, and more preferably type B.

### [Sensitizer]

The polymer to be used as the sensitizer for immunochromatographic assay of hepatitis virus as an assay target substance of the present invention is represented by the following general formula [1]: 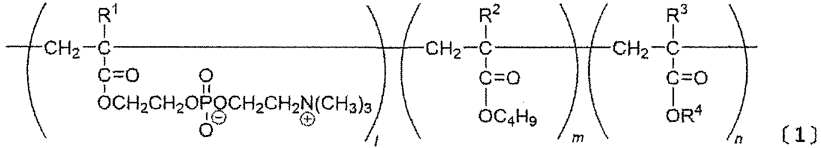 wherein R¹, R² and R³ are the same or different and each is a hydrogen atom or a methyl group, R⁴ is a hydrogen atom, a cation or a 2,3-dihydroxypropyl group, and 1, m and n are each a constitutional unit number and l:m:n is 20 to 100:0 to 80:0 to 80.

Examples of the cation include a sodium ion, a potassium ion, an ammonium ion and the like.

R¹, R² and R³ are preferably methyl groups.

R⁴ is preferably a hydrogen atom or a 2,3-dihydroxypropyl group.

The polymer represented by the above-mentioned general formula [1] is a water-soluble polymer having a weight-average molecular weight of 10,000 to 2,000,000.

In one embodiment, the polymer represented by the above-mentioned general formula [1] is composed of a polymer having only a constitutional unit based on a monomer represented by the following general formula [2] {MPC: 2-(methacryloyloxy)ethyl 2'-(trimethylammonio)ethyl phosphate}:

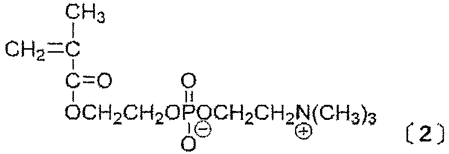

That is, the polymer is represented by the following general formula [3]: 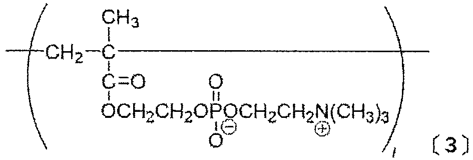 wherein 1 is a constitutional unit number.

The polymer represented by the above-mentioned general formula [3] is preferably a water-soluble polymer having a weight-average molecular weight of 10,000 to 2,000,000, more preferably 20,000 to 1,500,000.

The polymer represented by the above-mentioned general formula [3] may further contain other constitutional units as long as the effect of the present invention is not impaired. Examples of other constitutional unit include constitutional units based on monomers such as (meth)acrylic acids, alkyl (meth)acrylates, cyclic alkyl (meth)acrylates, aromatic group-containing (meth)acrylates, hydroxyl group-containing (meth)acrylates and the like. Among these monomers, (meth)acrylic acids, alkyl (meth)acrylates, and hydroxyl group-containing (meth)acrylates are preferred. The alkyl (meth)acrylate is preferably butyl methacrylate. The hydroxyl group-containing (meth)acrylate is preferably 2-hydroxyethyl(meth)acrylate or glycerol (meth)acrylate, particularly preferably glycerol (meth)acrylate. The other constitutional unit is particularly preferably a constitutional unit based on a monomer selected from butyl methacrylate, methacrylic acid and glycerol methacrylate.

In another embodiment, the polymer represented by the above-mentioned general formula [1] is composed of a copolymer having a constitutional unit based on MPC, and a constitutional unit based on butyl methacrylate.

That is, the polymer is represented by the following general formula [4]:

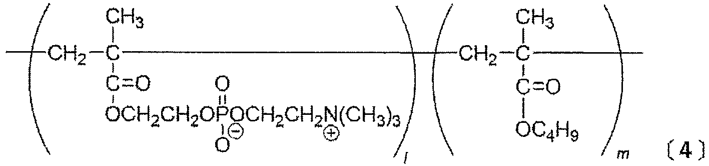

In the copolymer, the ratio of the constitutional unit number based on MPC and the constitutional unit number based on butyl methacrylate (l:m) is preferably 20 to 95:5 to 80, more preferably 25 to 85:15 to 75.

The copolymer is preferably a copolymer having a weight-average molecular weight of 10,000 to 1,000,000, more preferably 20,000 to 750,000.

The kind of the copolymer is not particularly limited, and may be a random copolymer or a block copolymer, with preference given to a random copolymer.

In another embodiment, the polymer represented by the above-mentioned general formula [1] is composed of a copolymer having a constitutional unit based on MPC, and a constitutional unit based on methacrylic acid.

That is, the polymer is represented by the following general formula [5]: 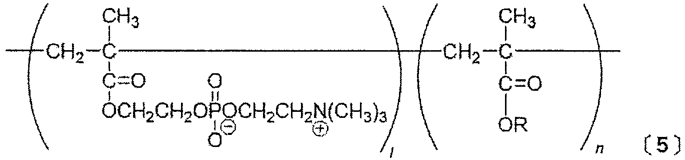 wherein R in the number of n are each independently a hydrogen atom or a cation.

In the copolymer, the ratio of the constitutional unit number based on MPC and the constitutional unit number based on methacrylic acid (l:n) is preferably 20 to 90:10 to 80, more preferably 25 to 85:15 to 75.

The copolymer is preferably a copolymer having a weight-average molecular weight of 10,000 to 1,000,000, more preferably 20,000 to 750,000.

The kind of the copolymer is not particularly limited, and may be a random copolymer or a block copolymer, with preference given to a random copolymer.

In another embodiment, the polymer represented by the above-mentioned general formula [1] is composed of a copolymer having a constitutional unit based on MPC, a constitutional unit based on butyl methacrylate, and a constitutional unit based on glycerol methacrylate.

That is, the polymer is represented by the following general formula [6]:

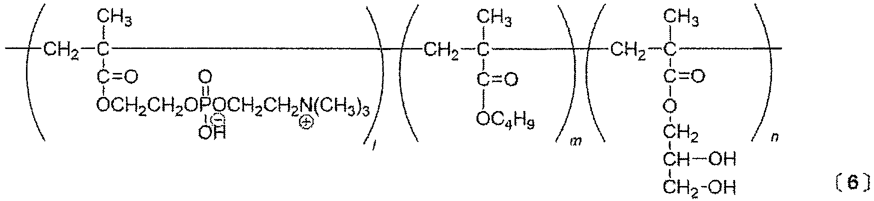

In the copolymer, the ratio of the constitutional unit number based on MPC, the constitutional unit number based on butyl methacrylate and the constitutional unit number based on glycerol methacrylate (l:m:n) is preferably 20 to 55:5 to 40:5 to 40, more preferably 25 to 45:15 to 45:15 to 45.

The copolymer is preferably a copolymer having a weight-average molecular weight of 10,000 to 1,000,000, more preferably 10,000 to 500,000.

The kind of the copolymer is not particularly limited, and may be a random copolymer or a block copolymer, with preference given to a random copolymer.

The polymer or copolymer to be used as the sensitizer for immunochromatographic assay is preferably exemplified by, but is not particularly limited to, those described in the following examples.

The polymer or copolymer to be used as the sensitizer for immunochromatographic assay of the present invention may be used by dissolving in various reagents used in immunochromatographic assay, or may be carried on an immunochromatographic assay kit. Preferably, it is used as a sample diluent.

In the immunochromatographic assay of the present invention, the immunochromatographic assay kit to be used usually contains or is composed of a sample diluent and a test strip.

The sample diluent is a diluent suitable for performing assay by immunochromatography, and may contain buffer components such as Tris buffer, phosphate buffer, Veronal buffer, borate buffer, and Good's buffer, stabilizing components such as albumin, globulin, casein, serum, water-soluble gelatin, surfactant, saccharides, and chelating agent, and preservative components such as salicylic acid, benzoic acid, and sodium azide.

When the sensitizer of the present invention is used by dissolving in a sample diluent, the concentration thereof during an antigen-antibody reaction is not particularly limited, but is usually used so that the net concentration is 0.005 w/v% or more and 10.0 w/v% or less, preferably 0.01 w/v% or more and 5.0 w/v% or less, particularly preferably 0.01 w/v% or more and 1.0 w/v% or less.

A test strip usually contains at least a first substance capable of recognizing a first antigenic determinant of an assay target substance in a sample and undergoing an antigen-antibody reaction, a labeled second substance capable of recognizing a second antigenic determinant of the aforementioned assay target substance and undergoing an antigen-antibody reaction, and a membrane carrier, and is configured such that the aforementioned first substance is immobilized in advance at a predetermined position on the aforementioned membrane carrier to form a trapping site, and the aforementioned second substance is disposed so as to be chromatographically developable on the aforementioned carrier at a position isolated from the aforementioned trapping site.

As the test strip for immunochromatographic assay, a commercially available product may be used or a test strip produced by a known method may also be used. When it is produced by a known method, the base material used in the above-mentioned development membrane may be those generally used in this field, and preferred examples thereof include cellulose, nitrocellulose, and nylon.

Examples of the instrument containing the sensitizer of the present invention which is for immunochromatographic assay of hepatitis virus as an assay target substance include, but are not particularly limited to, (1) one composed of a development membrane, (2) one composed of a sample-labeling part and a development membrane, and formed such that antibodies can move between the sample-labeling part and the development membrane by capillarity, (3) one composed of a sample-labeling part, a development membrane, and a liquid-absorbing part, and formed such that antibodies can move along the sample-labeling part, the development membrane, and the liquid-absorbing part in this order by capillarity, (4) one composed of a sample-dropping part, a sample-labeling part, a development membrane, and a liquid-absorbing part, and formed such that antibodies can move along the sample-dropping part, the sample-labeling part, the development membrane, and the liquid-absorbing part in this order by capillarity.

When the sensitizer of the present invention is used by being carried on the aforementioned instrument for immunochromatographic assay, the amount thereof varies depending on the location where it is carried, the kind of the sensitizer to be used, the kind of the assay target substance to be used, the labeling substance to be used, and the like. For example, when the sensitizer of the present invention is carried on areas such as the development membrane, the sample-labeling part, or the sample-dropping part of the instrument for immunochromatographic assay, the amount of the sensitizer contained per unit area (cm²) is usually 0.01 µg to 10 mg, preferably 0.1 µg to 4 mg, more preferably 1 to 800 µg. In addition, the area on which the sensitizer is carried varies depending on the kind and size of the instrument for immunochromatographic assay to be used and the amount of the assay sample. In the case of the development membrane of the instrument for immunochromatographic assay, it is usually 10 to 80%, preferably 10 to 60%, of the total test strip area, and in the case of the sample-dropping part, it is usually 5 to 40%, preferably 5 to 20%, of the total test strip area.

The present invention provides a test kit for hepatitis virus, containing the sensitizer for immunochromatographic assay of the present invention and a substance that specifically binds to hepatitis virus.

Being "specific" means that the affinity for hepatitis virus is higher than the affinity for other substances.

The aforementioned substance is preferably isolated or purified. The "isolated or purified" means that an operation has been performed to remove components other than the desired component from the natural state. The purity of the aforementioned isolated or purified substance (ratio of the weight of the aforementioned substance to the total protein weight) is usually 50% or more, preferably 70% or more, more preferably 90% or more, most preferably 95% or more (for example, substantially 100%).

The aforementioned substance may be directly or indirectly labeled with a labeling substance. Examples of the labeling substance include fluorescent substances (e.g., FITC, rhodamine), radioactive substances (e.g., ¹⁴C, ³H, ¹²⁵I), enzymes (e.g., alkali phosphatase, peroxidase), colored particles (e.g., metal colloid particles, colored latex), biotin, and the like.

In the present invention, examples of the "substance that specifically binds to hepatitis virus" include antibodies against hepatitis viruses, such as hepatitis A virus antibodies against hepatitis A virus, hepatitis B virus antibodies against hepatitis B virus, hepatitis C virus antibodies against hepatitis C virus, hepatitis D virus antibodies against hepatitis D virus, hepatitis E virus antibodies against hepatitis E virus, and the like. More specifically, the antibodies recognize and specifically bind to specific antigens of hepatitis viruses.

The aforementioned antigen is not limited as long as it is an antigen possessed by the hepatitis virus that is the assay target in the present invention, and for example, for hepatitis B virus, HBs antigen, HBc antigen, and HBe antigen are included, with HBs antigen being preferred. For hepatitis C virus, for example, HCV core antigen is included.

The aforementioned antibody is not limited as long as it is an antibody against the hepatitis virus that is the assay target in the present invention, and for example, for antibodies against hepatitis B virus, HBs antibody against HBs antigen, HBc antibody against HBc antigen (IgM-HBc antibody and IgG-HBc antibody), and HBe antibody against HBe antigen are included. For hepatitis C virus, an antibody against HCV core antigen is included.

The aforementioned antibody may be a polyclonal antibody, a monoclonal antibody, a chimera antibody, or a single-chain antibody. It may also be a part of an antibody having antigen-binding properties (antibody fragment), such as a Fab fragment or a fragment produced by a Fab expression library. Examples of the antibody fragment include, but are not limited to, (i) Fab fragment, which is a monovalent fragment consisting of VL, VH, CL, and CH1 domains, (ii) an F(ab')₂ fragment which is a divalent fragment containing two Fab fragments linked by a disulfide crosslinking at the hinge region, (iii) an Fv fragment consisting of the single-armed VL and VH domains of an antibody, (iv) a Fab' fragment obtained by breaking the disulfide crosslinking of the F(ab')₂ fragment by using mild reducing conditions, (v) an Fv fragment (dsFv) stabilized by disulfide, and (vi) a domain antibody (dAb) which is a single variable region domain (VH or VL) polypeptide of an antibody that specifically binds to an antigen.

The hepatitis virus antibody to be used in the method of the present invention may be produced by mouse, guinea pig, hamster, goat, or rabbit. As the hepatitis virus antibody, commercially available ones can be used.

The test kit of the present invention contains the above-mentioned sensitizer for immunochromatographic assay of the present invention and a substance that specifically binds to hepatitis virus in the same or separate containers.

The test kit of the present invention may further contain a manual stating that the sensitizer for immunochromatographic assay of the present invention and a substance that specifically binds to hepatitis virus can or should be used for testing for hepatitis virus.

The above-mentioned test kit for hepatitis virus can contain any carrier, for example, pharmaceutically acceptable carrier, stabilizer, buffer component, and the like. The pharmaceutically acceptable carrier includes, but is not limited to, a diluent such as water and physiological saline. The test kit for hepatitis virus of the present invention may contain a sample diluent, a test strip for immunochromatographic assay, and an instrument for immunochromatographic assay.

The present invention provides a method for testing a test subject for the possibility of being infected with hepatitis virus, including assaying hepatitis virus in a biological sample derived from the test subject by conducting an antigen-antibody reaction in the presence of the sensitizer for immunochromatographic assay of the present invention.

The biological sample to be subjected to the testing method of the present invention is a sample derived from a mammalian subject. The mammal is not particularly limited as long as it is a mammal that may be infected with a hepatitis virus. Among them, preferred are laboratory animals such as rodents (e.g., mice, rats, hamsters, guinea pigs, etc.) and rabbits; domestic animals such as pigs, cows, goats, horses, sheep, and minks; pets such as dogs, and cats; and primates such as humans, monkeys, cynomolgus monkeys, rhesus monkeys, marmosets, orangutans, and chimpanzees, and particularly preferred are humans.

The biological sample that can be used in the method is, for example, blood but is not particularly limited as long as the hepatitis virus can be detected. As the "blood", blood derived from any tissue can be assumed, but peripheral blood is generally used because of the easiness of collection. As a method for collecting blood, a method known per se can be applied. The collected blood may be used as it is in the present method, but may be used in the present method as a liquid component (plasma) obtained by separating cell components (erythrocyte, leukocyte, platelet, and the like) by a method known per se, such as centrifugation, filtration, and the like. It is also possible to use the blood in this method as a liquid component (serum) obtained by coagulating the blood and separating platelets and coagulation factors.

In conducting the test method of the present invention, for example, a biological sample such as blood derived from a test subject is suspended in an extraction liquid, and an antigen is extracted from the biological sample liquid. Then, when the above-mentioned instrument for immunochromatographic assay is used, the biological sample liquid treated with the extraction liquid is added dropwise onto the sample-dropping part. By doing so, the biological sample liquid is developed from the sample-dropping part to the sample-labeling part, and then onto the membrane, due to capillarity, in the presence of the sample diluent for immunochromatographic assay containing the sensitizer for immunochromatographic assay of the present invention.

The target antigen (e.g., hepatitis B virus) in the biological sample liquid (sample) forms an immunocomplex with a labeling substance (e.g., a first antibody against hepatitis B virus) carried on the sample-labeling part when passing through the sample-labeling part, and is developed as it is to one end of the development membrane. Then, the complex and the substance (e.g., a second antibody against hepatitis B virus) present on the test line of the development membrane (coated on the test line) cause an immunochemical reaction and bind to each other, and develop color, whereby a visible line appears on the development membrane. When the color develops in this way, the sample is positive, and when the target antigen in the biological sample liquid is hepatitis B virus, the test subject can be determined to be infected with the hepatitis B virus. In addition, the intensity of the positivity can be determined by visually observing or measuring the degree of coloration with an appropriate analytical instrument (e.g., immunochromatographic reader, spectrophotometer). For example, when a fluorescent dye is used as the detection means (labeling agent) that the labeled antibody has, UV or the like is irradiated on the test line, and whether the test line is positive or not can be determined based on the presence or absence of coloration and the degree of coloration.

On the other hand, a control line is provided after the test line, and an immunochemical reaction occurs between a labeling substance that has not yet formed an immunocomplex (e.g., a labeled antibody against hepatitis B virus antibody), and a substance that specifically binds to the aforementioned labeling substance (e.g., a secondary antibody against the aforementioned labeled antibody) present on the control line (coated thereon), causing binding and coloring, thereby making it possible to visually confirm that the immunochromatography was performed without any problems.

After the sample liquid has been developed and passed through the development membrane, the absorbent pad absorbs unreacted labeled antibody and the like. When the sample does not contain the target antigen (e.g., hepatitis B virus), the test line does not develop color and only the control line develops color. In this case, the sample is negative and, when the target antigen in the biological sample liquid is hepatitis B virus, the test subject is not determined to be infected with the hepatitis B virus.

### [Production method of the copolymer represented by the general formula [1]]

The copolymer represented by the general formula [1] can be obtained, for example, by polymerizing a monomer composition containing MPC which is a phosphorylcholine group-containing monomer represented by the above-mentioned formula [2] in a molar ratio of 20 or more and 100 or less, and other monomer as constitutional units in a molar ratio of 0 or more and 80 or less.

The polymerization reaction of the above-mentioned monomer composition can be carried out by replacing the reaction system with an inert gas such as nitrogen, carbon dioxide, argon etc., or under such an atmosphere, according to a known method such as radical polymerization, bulk polymerization, suspension polymerization, emulsion polymerization, solution polymerization etc. From the viewpoint of purification of the obtained polymer and the like, solution polymerization is preferred. The polymer of the present invention can be obtained by this polymerization reaction.

When these copolymers are purified, the purification can be carried out by general purification methods such as reprecipitation, dialysis, ultrafiltration, etc.

Radical polymerization initiators include azo radical polymerization initiators, organic peroxides, and persulfates.

Examples of the azo radical polymerization initiator include 2,2-azobis(2-aminopropane) dihydrochloride, 2,2-azobis(2-(5-methyl-2-imidazolin-2-yl)propane) dihydrochloride, 4,4-azobis(4-cyanovaleric acid), 2,2-azobisisobutylamide dihydrate, 2,2-azobis(2,4-dimethylvaleronitrile), 2,2-azobisisobutyronitrile (AIBN), dimethyl 2,2'-azobisisobutyrate, 1-((1-cyano-1-methylethyl)azo)formamide, 2,2'-azobis(2-methyl-N-phenylpropionamidine) dihydrochloride, 2,2'-azobis(2-methyl-N-(2-hydroxyethyl)propionamide), 2,2'-azobis(2-methylpropionamide) dihydrate, 4,4'-azobis(4-cyanopentanoic acid), 2,2'-azobis(2-(hydroxymethyl)propionitrile) and the like.

Examples of the organic peroxide include benzoyl peroxide, diisopropyl peroxydicarbonate, t-butyl peroxy-2-ethylhexanoate, t-butyl peroxypivalate, t-butyl peroxydiisobutyrate, lauroyl peroxide, t-butyl peroxyneodecanoate, succinic acid peroxide (=succinyl peroxide), glutaric peroxide, succinyl peroxyglutarate, t-butyl peroxymalate, t-butyl peroxypivalate, di-2-ethoxyethyl peroxycarbonate, 3-hydroxy-1,1-dimethylbutyl peroxypivalate, and the like.

Examples of the persulfate include ammonium persulfate, potassium persulfate, sodium persulfate and the like.

These radical polymerization initiators may be used alone or in combination. The amount of the polymerization initiator to be used is usually 0.001% by mass or more and 10% by mass or less, preferably 0.01% by mass or more and 5.0% by mass or less, relative to 100% by mass of the monomer composition.

The polymerization reaction of the above-mentioned monomer composition can be carried out in the presence of a solvent. As the solvent, a solvent that dissolves the monomer composition and does not react with the monomer composition before the addition of the polymerization initiator can be used. Examples thereof include water, alcohol solvents, ester solvents, ether solvents, and nitrogen-containing solvents. Examples of the alcohol solvent include methanol, ethanol, n-propanol, isopropanol and the like, examples of the ether solvent include ethyl cellosolve, tetrahydrofuran and the like, and examples of the nitrogen-containing solvent include acetonitrile, nitromethane, N-methylpyrrolidone and the like. The solvent is preferably water, alcohol, or mixed solvent thereof.

The temperature during the polymerization reaction is not limited and may be appropriately selected depending on the type of polymerization initiator and solvent to be used, and on the desired molecular weight, and is preferably in the range of 40°C or higher and 100°C or lower.

The present invention will be described in more detail below with reference to examples, but the present invention is not limited to these.

### [Example]

The polymers used in the present invention (Example) and Comparative Example are MPC polymer and random copolymers synthesized from MPC and the following monomers.

### (Polymer 1)

General formula [1] weight-average molecular weight; 1,030×10³, in the general formula [1], the monomer corresponding to 1 is MPC, and l:m:n is 100:0:0.

### (Polymer 2)

General formula [1] weight-average molecular weight; 600×10³, in the general formula [1], the monomer corresponding to 1 is MPC, the monomer corresponding to m is butyl methacrylate, and l:m:n is 80:20:0.

### (Polymer 3)

General formula [1] weight-average molecular weight; 93×10³, in the general formula [1], the monomer corresponding to 1 is MPC, the monomer corresponding to m is butyl methacrylate, and l:m:n is 30:70:0.

### (Polymer 4)

General formula [1] weight-average molecular weight; 550×10³, in the general formula [1], the monomer corresponding to 1 is MPC, the monomer corresponding to n is methacrylic acid, and l:m:n is 70:0:30.

### (Polymer 5)

General formula [1] weight-average molecular weight; 368×10³, in the general formula [1], the monomer corresponding to 1 is MPC, the monomer corresponding to n is methacrylic acid, and l:m:n is 30:0:70.

### (Polymer 6)

General formula [1] weight-average molecular weight; 22×10³, in the general formula [1], the monomer corresponding to 1 is MPC, the monomer corresponding to m is butyl methacrylate, the monomer corresponding to n is glycerol methacrylate, and l:m:n is 40:40:20.

### (Polymer 7)

General formula [1] weight-average molecular weight; 22×10³, in the general formula [1], the monomer corresponding to 1 is MPC, the monomer corresponding to m is butyl methacrylate, the monomer corresponding to n is glycerol methacrylate, and l:m:n is 40:20:40.

### <Measurement method of weight-average molecular weight>

The weight-average molecular weight means the value measured by gel permeation chromatography (GPC) in terms of PEG (polyethylene glycol), and the detailed measurement conditions are as follows.

### (Measurement conditions)

GPC system: High-speed GPC device HLC-8320 (manufactured by Tosoh Corporation)
column: OHpak SB-802.5 HQ and OHpak SB-806M HQ connected (manufactured by Showa Denko)
development solvent: 20 mM phosphate buffer at pH 7.4 detector: differential refractive index detector flow rate: 0.5 mL/min
injection volume: 10 µL
column temperature: 45°C
sample: The copolymer is diluted with a development solvent to a final concentration of 0.5 wt%.

In this example, it was confirmed whether the above-mentioned polymers can improve the detection sensitivity (have a sensitizing effect) of Quick Chaser (registered trademark) HBsAg (manufactured by Mizuho Medi Co., Ltd.), which is a known detection kit for hepatitis B virus. ACCURUN (registered trademark) series Infectrol (registered trademark) D-00 (manufactured by SeraCare Life Sciences Co., Ltd.) was used as a hepatitis B virus positive sample. Immunochromato Reader C10066-10 (manufactured by Hamamatsu Photonics Co., Ltd.) was used as a detector. Details are as follows.

### (Example 1)

A hepatitis B virus positive sample was diluted 1, 2, 4, 8, 16, and 32 times with a hepatitis B virus negative serum to prepare 360 µL of each solution. 40 µL of an aqueous solution containing 5.0 wt% of Polymer 1 was added to each solution and mixed.

### (Example 2)

The same procedure as in Example 1 was performed except that Polymer 2 was used instead of Polymer 1.

### (Example 3)

The same procedure as in Example 1 was performed except that Polymer 3 was used instead of Polymer 1.

### (Example 4)

The same procedure as in Example 1 was performed except that Polymer 4 was used instead of Polymer 1.

### (Example 5)

The same procedure as in Example 1 was performed except that Polymer 5 was used instead of Polymer 1.

### (Example 6)

The same procedure as in Example 1 was performed except that Polymer 6 was used instead of Polymer 1.

### (Example 7)

The same procedure as in Example 1 was performed except that Polymer 7 was used instead of Polymer 1.

### (Comparative Example 1)

The same procedure as in Example 1 was performed except that distilled water was used instead of Polymer 1.

### [Evaluation method]

The sample prepared in each Example and Comparative Example was evaluated as follows.

### (Confirmation of hepatitis B virus detection sensitivity using a known instrument for immunochromatographic assay)

100 µL of the sample prepared in each Example and Comparative Example was dropped onto the sample-dropping part of a detection kit for hepatitis B virus. After 15 minutes, the coloration intensity of the test line was detected with an immunochromato reader. When the evaluation was performed three times using negative serum as a sample, the average coloration intensity of the test line was 3.67 mABS. Therefore, when the measured detection sensitivity was greater than 3.67 mABS, it was judged as "positive", and when it was 3.67 mABS or less, it was judged as "negative". For each Example and Comparative Example, if the positive sample was "positive" at a dilution of 1 time or greater and 16 times or 32 times or less, it was evaluated as excellent (⊚). If the positive sample was "positive" at a dilution of 1 time or greater and 8 times or less, it was evaluated as good (○). If the positive sample was "positive" at a dilution of 1 time or greater and 4 times or less, it was evaluated as fair (△). If the positive sample was "positive" at a dilution of 1 time or greater and 2 times or less, it was evaluated as unacceptable (×).

### (Confirmation results of hepatitis B virus detection sensitivity using an instrument for immunochromatographic assay)

The results of improved detection sensitivity using a detection kit for hepatitis B virus are shown in Table 1.

**Table 1**

| Example | | 1 | 2 | 3 | 4 | 5 | 6 | 7 | Comparative Example 1 |
|---|---|---|---|---|---|---|---|---|---|
| Dilution rate | | | | | | | | | |
| Measured value (mABS) | x1 | 146.07 | 220.50 | 221.41 | 189.16 | 207.68 | 114.64 | 110.01 | 87.26 |
| | x2 | 45.58 | 59.46 | 62.85 | 93.89 | 112.91 | 27.43 | 26.13 | 17.06 |
| | x4 | 9.94 | 14.01 | 9.96 | 36.44 | 40.22 | 6.66 | 5.57 | 2.90 |
| | x8 | 5.85 | 4.43 | 1.65 | 8.23 | 10.43 | 2.62 | 2.68 | 1.90 |
| | x16 | 3.30 | 0.92 | 0.93 | 5.88 | 6.85 | 2.46 | 2.11 | 1.60 |
| | x32 | 2.33 | 2.29 | 1.53 | 3.34 | 3.90 | 0.68 | 0.74 | 1.56 |
| Judgement | x1 | **positive** | **positive** | **positive** | **positive** | **positive** | **positive** | **positive** | **positive** |
| | x2 | **positive** | **positive** | **positive** | **positive** | **positive** | **positive** | **positive** | **positive** |
| | x4 | **positive** | **positive** | **positive** | **positive** | **positive** | **positive** | **positive** | negative |
| | x8 | **positive** | **positive** | **negative** | **positive** | **positive** | **negative** | **negative** | negative |
| | x16 | **negative** | **negative** | **negative** | **positive** | **positive** | negative | negative | negative |
| | x32 | negative | negative | negative | negative | **positive** | negative | negative | negative |
| Evaluation | | ⊚ | ⊚ | ○ | ⊚ | ⊚ | ○ | ○ | Δ |

When Examples 1 to 7 and Comparative Example 1 were added, the evaluation of Comparative Example 1 for hepatitis B virus was fair (△), whereas the evaluation of Examples 1 to 7 was excellent (⊚) or good (○), confirming that the sensitivity was improved.

As can be seen from the above examples, the present invention can provide an immunochromatographic assay with a significantly superior sensitivity enhancement effect compared to known immunochromatographic assays of hepatitis virus as an assay target substance.

### [Industrial Applicability]

The present invention can provide a sensitizer for immunochromatographic assay of hepatitis virus as an assay target substance, a sample diluent for immunochromatographic assay, immunochromatographic assay, an instrument for immunochromatographic assay, and a test kit for hepatitis B virus.

This application is based on a patent application No. 2023-018698 filed in Japan (filing date: February 9, 2023), the contents of which are incorporated in full herein.

## Claims

1. A sensitizer for immunochromatographic assay of hepatitis virus as an assay target substance, comprising a polymer represented by the following general formula [1]: 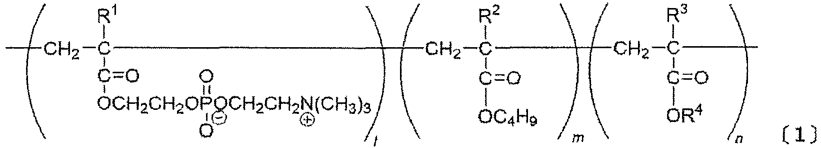 wherein R¹, R² and R³ are the same or different and each is a hydrogen atom or a methyl group, R⁴ is a hydrogen atom, a cation or a 2,3-dihydroxypropyl group, and 1, m and n are each a constitutional unit number and l:m:n is 20 to 100:0 to 80:0 to 80.

2. A sample diluent for immunochromatographic assay of hepatitis virus as an assay target substance, comprising the sensitizer for immunochromatographic assay according to claim 1.

3. A method for immunochromatographically assaying hepatitis virus as an assay target substance, comprising conducting an antigen-antibody reaction in the presence of the sensitizer for immunochromatographic assay according to claim 1.

4. An instrument for immunochromatographic assay of hepatitis virus as an assay target substance, comprising the sensitizer for immunochromatographic assay according to claim 1.

5. A test kit for hepatitis virus, comprising a polymer represented by the following general formula [1] and a substance that specifically binds to hepatitis virus: 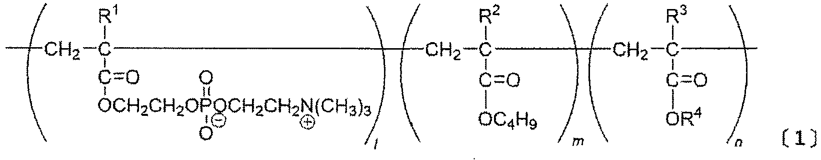 wherein R¹, R² and R³ are the same or different and each is a hydrogen atom or a methyl group, R⁴ is a hydrogen atom, a cation or a 2,3-dihydroxypropyl group, and 1, m and n are each a constitutional unit number and l:m:n is 20 to 100:0 to 80:0 to 80.
